Europäisches Patentamt

European Patent Office

Office européen des brevets

(19)

(11) Numéro de publication : **0 605 279 A1**

## (12) DEMANDE DE BREVET EUROPEEN

(21) Numéro de dépôt : 93403061.0

(22) Date de dépôt : 16.12.93

(51) Int. Cl.⁵ : **B01J 31/02,** B01J 27/053,
C07C 2/62

(30) Priorité : 22.12.92 FR 9215696

(43) Date de publication de la demande :
06.07.94 Bulletin 94/27

(84) Etats contractants désignés :
BE DE FR GB IT NL SE

(71) Demandeur : INSTITUT FRANCAIS DU
PETROLE
4, Avenue de Bois Préau
F-92502 Rueil-Malmaison (FR)

(72) Inventeur : Joly, Jean-Francois
20 Rue Béranger
F-75003 Paris (FR)
Inventeur : Marcilly, Christian
91 ter rue Condorcet
F-7880 Houilles (FR)
Inventeur : Ferrer, Nathalie
34 avenue Guy de Maupassant
F-78400 Chatou (FR)

(54) Catalyseur d'alkylation de paraffines.

(57) Catalyseur à base d'un support poreux minéral ou organique et du mélange équimolaire constitué par les acides trifluorométhanesulfonique et sulfurique anhydres, et son utilisation en alkylation catalytique d'isobutane et/ou d'isopentane en présence d'au moins une oléfine comportant de 3 à 6 atomes de carbone par molécule.

EP 0 605 279 A1

La présente invention concerne un catalyseur à base d'un support poreux minéral ou organique et du mélange équimolaire constitué par les acides trifluorométhanesulfonique et sulfurique anhydres, et son utilisation en alkylation catalytique d'isobutane et/ou d'isopentane au moyen d'une oléfine, qui permet d'obtenir au moins un produit par exemple dans le groupe constitué par les diméthylbutanes, les triméthylpentanes, les triméthylhexanes et les triméthylheptanes.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation des isoparaffines (isobutane et/ou isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes. Parmi les familles de catalyseurs acides on peut citer les tamis moléculaires (voir par exemple US-A-3.236.762, US-A-3.251.902, US-A-3.644.565, US-A-4.377.721, US-A-4.384.161 et US-A-4.300.015), les résines macroréticulaires éventuellement associées avec $BF_3$ (voir par exemple US-A-3.855.342, US-A-3.855.343, US-A-3.862.258 et US-A-3.879.489), les résines perfluorées de type NAFION (voir par exemple US-A-4.056.578 et US-A-4.038.213), les acides de Lewis et/ou de Bronsted déposés sur divers supports inorganiques (voir par exemple US-A-3.975.299, US-A-3.852.371 et US-A-3.979.476), les alumines chlorées (voir par exemple US-A-3.240.840, US-A-3.523.142, US-A-3.607.859, US-A-3.523.142, US-A-4.066.716, US-A-4.083.800 et US-A-4.066.716), les graphites intercalés par des acides de Lewis et/ou de Bronsted (voir par exemple US-A-4.083.885, US-A-4.116.880, US-A-4.128.596 et US-A-3.976.714) et les anions déposés sur supports oxydes tels que $ZrO_2/SO_4$ (voir par exemple J-01288329, J-011245854-A, J-01245953, J-61242641-A et J-61242641). Ces solides conduisent à la production d'isoparaffines ramifiées mais souffrent de plusieurs défauts majeurs, parmi lesquels on peut citer l'utilisation de rapports molaires isobutane/oléfine souvent très élevés pour limiter l'importance des réactions secondaires et la faible stabilité dans le temps de l'activité catalytique (inhibition du catalyseur par dépôt d'oligomères insaturés) ; ces catalyseurs doivent alors être fréquemment régénérés. De plus, la faible acidité de certains solides acides, tels que les tamis moléculaires par exemple, impose l'utilisation de températures de réaction élevées ce qui est préjudiciable à l'obtention d'hydrocarbures d'indices d'octane élevés.

La demande de brevet EP-A-0433954 revendique l'utilisation d'acides fluorosulfoniques, dont les acides $FSO_3H$ et $CF_3SO_3H$, pour la réaction d'alkylation de l'isobutane par les oléfines en lit fixe. Les auteurs montrent, dans un exemple dudit brevet, que l'acide sulfurique mis en oeuvre dans les conditions selon ladite invention conduit à de très mauvais résultats.

La demande de brevet EP-A-0.539.277 décrit un catalyseur comprenant de la silice et de l'acide sulfurique à l'état solide, la silice étant imprégnée par une solution contenant de l'acide sulfurique et éventuellement par un additif dont l'acide trifluorométhanesulfonique $CF_3SO_3H$.

La présente invention concerne un catalyseur de composition particulière permettant d'obtenir des composés paraffiniques à hauts degrés de ramification et hauts indices d'octane par alkylation de l'isobutane et/ou de l'isopentane avec des oléfines comprenant de 3 à 6 atomes de carbone par molécule. Ce catalyseur est avantageusement mis en oeuvre dans un procédé où l'oléfine et/ou un mélange d'oléfines est introduit(e) dans le réacteur en phase liquide et en mélange avec l'isoparaffine et/ou un mélange d'isoparaffines.

Le catalyseur de la présente invention renferme un support poreux minéral, de préférence la silice, ou organique et le mélange équimolaire constitué par les acides trifluorométhanesulfonique et sulfurique anhydres, ledit support poreux minéral ou organique étant imprégné partiellement ou totalement par ledit mélange. Dans le cas où la silice est utilisée comme support, elle peut contenir des impuretés comme par exemple les oxydes, les alcalins, les alcalinoterreux, les composés d'aluminium ou toute autre impureté connue de l'homme du métier, la quantité totale de ces impuretés n'excédant pas 2 % en poids par rapport à la silice. La surface spécifique du support poreux minéral ou organique doit être comprise entre 0,01 et 1500 $m^2/g$, de préférence entre 0,01 et 150 $m^2/g$ et de manière souvent plus préférée entre 0,01 et 50 $m^2/g$. Le volume poreux total du support minéral ou organique doit être compris dans l'intervalle de valeurs suivant: entre 0,005 et 3 cm³/g, de préférence entre 0.005 et 1.5 cm³/g, et de manière préférée entre 0,005 et 1 cm³/g. D'une façon préférée, ledit poreux support minéral ou organique est constitué de grains sensiblement sphériques de diamètre moyen compris entre 5 et 900μm, de préférence entre 5 et 150μm, de manière préférée entre 5 et 110 μm et de manière encore plus préférée entre 5 et 70 μm.

Lors de l'imprégnation du support poreux minéral ou organique, la solution constituée du mélange équimolaire constitué par les acides trifluorométhanesulfonique et sulfurique anhydres occupe généralement une fraction du volume poreux total comprise entre 5 et 100 %, de préférence comprise entre 80 et 100 % et de manière encore plus préférée entre 90 et 100 %. Le catalyseur ainsi obtenu est caractérisé par une surface spécifique comprise entre 0,01 et 500 m²/g, de préférence entre 0,01 et 150 cm²/g et de manière encore plus préférée entre 0,01 et 40 m²/g.

Le procédé de préparation du catalyseur selon l'invention comprend généralement les trois étapes décrites ci-après, l'ordre des deux premières étapes pouvant être inversé.

- Dans une première étape, le mélange équimolaire constitué par les acides trifluorométhanesulfonique et sulfurique anhydres est préparé par addition lente et sous agitation d'acide trifluorométhane sulfonique anhydre dans la totalité de l'acide sulfurique anhydre. La durée totale d'injection de l'acide trifluorométhanesulfonique est habituellement comprise en quelques minutes et 1 heure. La préparation de ce mélange, ainsi que sa conservation, doivent être effectuées à l'abri de l'humidité.

- Dans une seconde étape le support poreux minéral ou organique est calciné à une température supérieure à 50 °C, de préférence supérieure à 80 °C et de manière encore plus préférée comprise entre 200 et 1000°C, par exemple égale à environ 500 °C. La durée de cette étape de calcination est habituellement comprise entre 10 minutes et 50 heures. La calcination peut être effectuée en présence d'air ou de mélange air/azote. de débit compris entre 0,001 et 10 l/h/g.

- La troisième étape consiste en l'imprégnation du support poreux minéral ou organique calciné par la solution constituée par le mélange équimolaire constitué par les acides trifluorométhanesulfonique et sulfurique anhydres. Pour réaliser cette étape on peut utiliser toutes les techniques bien connues de l'homme du métier. Conservé à l'abri de l'humidité, le catalyseur selon la présente invention est ainsi constitué d'un support poreux minéral ou organique imprégné par le mélange équimolaire constitué par les acides trifluorométhanesulfonique et sulfurique anhydres.

La température de réaction est généralement inférieure à +10° C, de prérérence comprise entre -30 et +5° C, de manière préférée entre -15 et 0° C. La pression du réacteur est suffisante pour maintenir les hydrocarbures à l'état liquide dans le réacteur.

Le mélange isoparaffine(s)-oléfine(s) peut être introduit dans le réacteur à une vitesse spatiale horaire, exprimée en poids d'oléfine introduite par unité de poids du catalyseur et par heure comprise entre 0,001 et 10 h⁻¹ et de préférence comprise entre 0,002 et 2 h⁻¹. Ledit mélange peut aussi être réalisé à l'intérieur du réacteur. Dans tous les cas le mélange ainsi constitué est dans le réacteur dans des conditions de pression et de température telles que le mélange d'hydrocarbures reste liquide sur le catalyseur.

Afin de limiter les réactions secondaires, on peut utiliser un excès d'isoparaffine par rapport à l'oléfine. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butènes dans la charge est généralement compris entre 1 et 100, de préférence entre 3 et 50 et de manière souvent préférée entre 5 et 15.

Le catalyseur selon la présente invention, permet de procéder à l'alkylation de l'isobutane et/ou de l'isopentane à basse température, ce qui permet de limiter les réactions secondaires et en particulier l'oligomérisation des oléfines.

Avec le catalyseur selon l'invention constitué d'un support poreux minéral ou organique contenant le mélange équimolaire constitué par les acides trifluorométhanesulfonique et sulfurique anhydres, les sélectivités en alkylation de l'isobutane par les butènes sont très nettement supérieures à celles obtenues, en utilisant le même procédé, avec un catalyseur contenant un même support imprégné par de l'acide sulfurique. De plus, la stabilité, donc la productivité, du catalyseur selon l'invention sont nettement améliorées par rapport au catalyseur contenant de l'acide sulfurique.

Lorsque les conditions de travail du catalyseur sont judicieusement choisies (en particulier la température), le catalyseur selon l'invention permet la production de produits d'alkylation des paraffines par les oléfines qui sont intéressants comme carburants pour les moteurs et constituants d'essence et qui comprennent par exemple au moins 90 % moles de paraffines possédant 8 atomes de carbone par molécule et moins de 1 % moles de composés non saturés, les paraffines comprenant 8 atomes de carbone par molécule comprenant 95 à 98 % en moles de triméthylpentanes.

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

### Exemple 1

#### Préparation d'un catalyseur selon l'invention : catalyseur A

On active 14 g de silice macroporeuse de surface spécifique égale à 55 m²/g de volume poreux total égal à 1,2 cm³/g et des grains sensiblement sphériques de diamètre moyen égal à 110 µm, par calcination sous air pendant 4 heures à 500°C. La silice ainsi activée est conservée sous argon.

Dans un récipient de type Erlen Meyer on introduit, sous débit d'argon sec, successivement et sous agitation 6,92 g d'acide sulfurique anhydre (soit 0,0707 mole) et 10,6 g d'acide trifluorométhanesulfonique anhydre (soit 0,0707 mole). L'acide trifluorométhanesulfonique anhydre est ajouté en 5 minutes. Le mélange ainsi obtenu est homégénéisé sous agitation pendant 1 heure.

On procède alors à une imprégnation à sec de 12,6 g de ladite silice par 17,4 g du mélange équimolaire constitué par les acides sulfurique et trifluorométhanesulfonique anhydres. Le solide ainsi obtenu est conservé sous argon jusqu'à utilisation.

#### Préparation d'un catalyseur comparatif : catalyseur B

Le catalyseur est préparé en effectuant une imprégnation à sec de 15 g d'une silice caractérisée par un volume poreux total de 1,2 cm³/g, une surface spécifique égale à 55 m²/g et des grains sensiblement sphériques de diamètre moyen égal à 110µm, et ayant été calcinée sous air à 500°C pendant 4 heures, par 12 cm³ d'une solution d'acide sulfurique à 96,5% en poids. Le catalyseur ainsi obtenu contient 15 g de silice et 22 g d'acide sulfurique et est conservé à l'abri de l'air jusqu'à utilisation.

### Exemple 2

#### Conditions de test catalytique d'alkylation de l'isobutane

Pour alkyler l'isobutane par le butène-1, on utilise un réacteur tubulaire de diamètre interne égal à 2 cm et de hauteur égale à 30 cm. Ce réacteur, qui contient la totalité de l'un ou l'autre des catalyseurs préparé selon l'exemple 1, est alimenté par le bas par une phase liquide dont la vitesse linéaire, dans le réacteur vide est égale à 1,6 mm/sec, vitesse suffisante pour assurer la fluidisation du catalyseur (la vitesse minimum de fluidisation de ce catalyseur est voisine 0,1 mm/sec). En sortie du réacteur, les effluents liquides sont envoyés dans une zone de séparation gaz-liquide d'un volume égal à 85 cm³. Une pompe de type Lewa (pompe à membrane) prélève du liquide de ce séparateur gaz-liquide pour alimenter le réacteur, le débit de la pompe est de 1,8 l/h. Un mélange d'isobutane et de butène-1, contenant 11,5% en poids de butène-1, est continuement injecté dans la partie inférieure du séparateur gaz-liquide avec un débit qui correspond à 0,04 g de butène-1 par gramme de catalyseur et par heure. On extrait en continu, de ce même séparateur une phase liquide composée d'isobutane et d'alkylat à un débit égal au débit d'injection de la charge isobutane-butène-1, cette phase liquide est introduite dans un second séparateur gaz liquide duquel on extrait en tête du séparateur un gaz contenant environ 98% en poids d'isobutane et en bas du séparateur un liquide constitué d'isoparaffines de 5 à 12 atomes de carbone par molécule que l'on appelle alkylat. L'agitation au sein du réacteur se fait grâce à un barreau magnétique.

La température, mesurée au sein du lit de catalyseur est égale à -2°C, la pression du réacteur est égale à 5 bar.

#### Résultats

La durée totale du test catalytique est de 24 heures, la composition pondérale des l'alkylats récupérés, selon que l'on utilise l'un ou l'autre des catalyseurs préparés selon l'exemple 1 est indiquée dans le tableau 1 ci-après :

Tableau 1

| | Catalyseur (invention) A | Catalyseur (comparatif) B |
|---|---|---|
| Conversion du butène-1 | 100% | 100% |
| $C_5$-$C_7$ | 1,75 % | 15,1 % |
| $C_8$ | 96,6 % | 60,5 % |
| $C_9^+$ | 1,65 % | 24,4 % |
| TMPs/$C_8$ | 95,8 % | 86,0 % |

TMPs/$C_8$ : teneur en triméthylpentanes dans la coupe $C_8$.

Le catalyseur selon la présente invention est nettement plus sélectif vis-à-vis de la production de composés à 8 atomes de carbone par molécule et plus particulièrement des triméthylpentanes dont les indices d'octane sont les plus élevés.

**Revendications**

1.  Catalyseur comprenant un support poreux minéral ou organique et le mélange équimolaire constitué par les acides trifluorométhanesulfonique et l'acide sulfurique anhydres, le support poreux minéral ou organique étant imprégné par le mélange équimolaire constitué par les acides trifluorométhanesulfonique et sulfurique anhydres et ayant avant imprégnation une surface spécifique comprise entre 0,01 et 1500 $m^2$/g, un volume poreux total compris entre 0.005 et 3 $cm^3$/g.

2.  Catalyseur selon la revendication 1 tel que ledit support est principalement constitué de grains sensiblement sphériques de diamètre moyen compris entre 5 et 900µm.

3.  Catalyseur selon l'une des revendications 1 ou 2 tel que ledit support est principalement constitué de grains sensiblement sphériques de diamètre moyen compris entre 5 et 150µm.

4.  Procédé de préparation d'un catalyseur selon l'une des revendications 1 à 3 tel que l'on prépare le mélange équimolaire constitué par les acides trifluorométhanesulfonique et sulfurique anhydres, puis le support minéral ou organique est calciné et enfin l'on imprègne ledit support par ledit mélange.

5.  Procédé selon la revendication 4 tel que ledit mélange occupe lors de l'imprégnation une fraction du volume poreux comprise entre 80 et 100 %.

6.  Utilisation du catalyseur selon l'une des revendications 1 à 3 ou préparé selon l'une des revendications 4 ou 5 dans un procédé d'alkylation dans lequel on traite une charge comprenant d'une part au moins une isoparaffine et d'autre part au moins une oléfine contenant de 3 à 6 atomes de carbone par molécule.

7.  Utilisation selon la revendication 6 dans lequel l'isoparaffine est choisie dans le groupe formé par l'isobutane et l'isopentane.

8.  Utilisation selon la revendication 7 telle que la température de la réaction est inférieure à 10° C.

9. Utilisation selon l'une des revendications 7 ou 8 telle que la température de réaction est comprise entre -15 et 0° C.

EP 0 605 279 A1

**Office européen des brevets**

# RAPPORT DE RECHERCHE EUROPEENNE

Numero de la demande

EP 93 40 3061

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | US-A-4 038 212 (BROCKINGTON ET AL)<br>* abrégé *<br>* colonne 3, ligne 8 - ligne 63 *<br>--- | 1 | B01J31/02<br>B01J27/053<br>C07C2/62 |
| D,Y<br><br>A | EP-A-0 433 954 (HALDOR TOPSOE)<br>* abrégé; figure 1; exemple 1 *<br>* page 4, ligne 3 - ligne 29; revendications *<br>--- | 1<br><br>2,4 | |
| A | US-I-383 581 (PARKER ET AL)<br>--- | | |
| D,P,<br>X | EP-A-0 539 277 (IFP)<br><br>* colonne 2, ligne 32 - colonne 3, ligne 6; revendications 1-9 *<br>--- | 1 | |
| P,X | FR-A-2 683 739 (IFP)<br>* page 3, ligne 14 - ligne 34; revendications *<br>----- | 1 | |

**DOMAINES TECHNIQUES RECHERCHES (Int.Cl.5)**

B01J
C07C

Le présent rapport a été établi pour toutes les revendications

| Lieu de la recherche | Date d'achèvement de la recherche | Examinateur |
|---|---|---|
| LA HAYE | 28 Mars 1994 | Lo Conte, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

7